# EUROPÄISCHE PATENTSCHRIFT

(11) **EP 0 336 146 B1**
(45) Veröffentlichungstag und Bekanntmachung des Hinweises auf die Patenterteilung: **11.08.1993**
(21) Anmeldenummer: 89104495.0
(22) Anmeldetag: 14.03.1989
(51) Int. Cl.: C07C 229/56

(54) **Verfahren zur Herstellung des 2-(2-Hydroxyethoxy)ethanolesters von Flufenaminsäure**
Process for the preparation of the 2-(2-hydroxy-ethoxy)ethanol ester of flufenaminic acid
Procédé de préparation de l'ester 2-(2-hydroxy-éthoxy)éthanolique de l'acide flufénaminique

(30) Priorität: 31.03.1988 DE 3811119
(43) Veröffentlichungstag der Anmeldung: 11.10.1989
(73) Patentinhaber: Merckle GmbH, D-89143 Blaubeuren (DE)
(72) Erfinder: Metz, Gunter, Dr., D-7902 Blaubeuren (DE)
(74) Vertreter: Lederer, Franz, Dr.

(56) Entgegenhaltungen:
- DE-A- 2 834 167
- DE-A- 2 834 170
- DE-B- 2 834 167

## Beschreibung

Die vorliegende Erfindung betrifft ein neues, verbessertes Verfahren zur Herstellung des bekannten 2-(2-Hydroxyethoxy)ethyl-N-(α,α,α- trifluor-m-tolyl)-anthranilats(I) (2-(2-Hydroxyethoxy)ethanolester von Flufenaminsäure).

Aus der DE-PS 19 39 112 ist bekannt, daß I durch Umsetzung des Kaliumsalzes der N-(α,α,α -Trifluor-m-tolyl)anthranilsäure(II) (Flufenaminsäure) mit 2-(2-Chlorethoxy)ethanol in Dimethylformamid hergestellt werden kann. Dieses Verfahren ist im technischen Maßstab jedoch nur unbefriedigend nutzbar und zeigt erhebliche Nachteile bei der Synthese, der Aufarbeitung und der Reinheit des Endproduktes.

Ein Verfahren, das diese Nachteile umgeht, ist in der DE-PS 28 34 167 beschrieben. Hiernach wird II mit überschüssigem 2-(2-Hydroxyethoxy)ethanol(III) in inerten Lösungsmitteln oder ohne Lösungsmittel mittels saurer Katalyse direkt umgesetzt, wobei das bei der Veresterung entstehende Reaktionswasser azeotrop entfernt wird. Anschließend wird nach diesem Verfahren das überschüssige III in Vakuum abdestilliert, der Rückstand in Diisopropylether aufgenommen, mit Natronlauge, Wasser oder Kochsalzlösung gewaschen, erneut im Vakuum eingedampft und danach der Ester I einer Molekulardestillation unterworfen. Gemäß dem Verfahren der DE-PS 28 34 167 wurden nach 16-stündiger Reaktionszeit bei 140 °C, sowie Verwendung von p-Toluolsulfonsäure als Katalysator, etwa 69 % I, bei Verwendung von Schwefelsäure als Katalysator, sowie 10-stündiger Reaktion bei 140 °C, etwa 36 % I erzielt.

Nach einem alternativen Herstellungsverfahren gemäß DE-PS 28 34 168 wird zunächst Flufenaminsäure (II) mit einem C₁-C₅-Alkohol unter azeotroper Entfernung des entstehenden Wassers verestert und der so entstandene niedermolekulare Ester mit 2-(2-Hydroxyethoxy)ethanol(III) umgeestert.

Es wurde nun überraschend gefunden, daß die sauer katalysierte direkte Veresterung der Säure II mit dem Diol III ohne azeotropes Auskreisen des Reaktionswassers, in wesentlich kürzeren Reaktionszeiten und auch bei niedrigeren Temperaturen mit Ausbeuten an reinem I von 60-70 % (Rohprodukt 80-90 %) durchgeführt werden kann, wenn ein erheblicher Überschuß an dem Diol III eingesetzt wird.

Hierbei überrascht besonders, daß das Verbleiben des Reaktionswassers im Ansatz eine derart hohe Esterausbeute erlaubt, da nach gängiger Lehrmeinung, auf der auch die DE-PS 28 34 167 aufbaut, ein Verschieben des Reaktionsgleichgewichtes zugunsten des Esters Entfernung des Reaktionswassers, d.h. azeotropes Auskreisen erfordert.

Das erfindungsgemäße Verfahren zur Herstellung des 2-(2-Hydroxyethoxy)ethanolesters von Flufenaminsäure (N-(α,α,α-Trifluor-m-tolyl)anthranilsäure) durch direkte Veresterung von Flufenaminsäure mit einem Überschuß an 2-(2-Hydroxyethoxy)ethanol bei Reaktionstemperaturen von 100 bis 140 °C, vorzugsweise 100 bis 125 °C und besonders bevorzugt 100 bis 120 °C in Gegenwert saurer Katalysatoren ist dadurch gekennzeichnet, daß man die Umsetzung mit einem Überschuß an dem Diol von 8-15 Mol je Mol Flufenaminsäure ohne gleichzeitige Entfernung des Reaktionswassers aus dem Reaktionsansatz durchführt.

Die erfindungsgemäße Reaktion erfolgt in Gegenwart von sauren Katalysatoren, wie z.B. Lewissäuren, Zinkchlorid, Schwefelsäure, Phosphorsäure, Benzolsulfonsäure, p-Toluolsulfonsäure, insbesondere p-Toluolsulfonsäure-Monohydrat oder Gemischen aus Phosphorsäure und p-Toluolsulfonsäure, bei molaren Verhältnissen von 0,25 bis 1 Mol, bevorzugt 0,5 bis 1 Mol Katalysator je Mol Säure II. Bevorzugt werden Schwefelsäure oder p-Toluolsulfonsäure verwendet. Die Reaktionstemperatur beträgt bevorzugt 100 °C bis 125 °C und besonders bevorzugt 100 bis 120 °C.

Höhere Temperaturen erhöhen nicht die Ausbeute, lediglich bei der Katalysatormischung aus Phosphorsäure und p-Toluolsulfonsäure war bei 140 °C eine Erhöhung der Esterausbeute um 5-20 % festzustellen. Bei Verwendung eines Gemisches aus Phosphorsäure und p-Toluolsulfonsäure werden bevorzugt Katalysatormengen zwischen 0,5 und 1 Mol eingesetzt. In diesem Fall kann die Verlängerung der Reaktionszeit auf 8 Stunden erforderlich werden, wenn eine Reaktionstemperatur von 120 °C gewählt wird.

Die Reaktionszeit beträgt 2 bis 4 Stunden bei Verwendung von 0,5 bis 1 Mol Katalysator, insbesondere bei Schwefelsäure oder p-Toluolsulfonsäure, je Mol Säure II und ist lediglich bei Katalysatormengen kleiner als 0,5 Mol je Mol Säure II auf 6 bis maximal 8 Stunden zu erhöhen. Bevorzugt werden Reaktionszeiten von bis zu 6 Stunden.

Auf je 1 Mol der Säure II werden 8 bis 15 Mol Diol III, vorzugsweise 10,5 bis 12 Mol eingesetzt.

Der Überschuß an Diol III wird gemäß dem erfindungsgemäßen Verfahren vorteilhaft ohne Destillation aus dem Reaktionsansatz entfernt. Dies erfolgt entweder durch Neutralisieren bzw. schwaches Alkalisieren des Ansatzes mit wäßrigen Lösungen von Alkali- oder Erdalkalihydroxyden oder -carbonaten, wobei sich der Rohester I abscheidet und Überschüssiges Diol in die wäßrige Phase überführt wird, oder durch Verrühren des Reaktionsansatzes, vorzugsweise bei einer Temperatur von 20-60 °C mit festem Alkali- oder Erdalkalihydroxyd oder -carbonat und direkte Extraktion des Rohesters I mit Kohlenwasserstoffen, wie z.B. Toluol.

Der erhaltene Rohester I kann direkt einer zweistufigen Vakuum-Kurzwegdestillation zugeführt werden, wobei in der ersten Stufe Lösungsmittelreste entfernt werden und in der zweiten Stufe der reine Ester I destillativ von höhermolekularen Verunreinigungen, insbesondere dem bei der Reaktion und thermisch bei der Destillation gebildeten Diester, abgetrennt wird. Ein weiteres besonders schonendes Aufarbeitungsverfahren besteht darin, daß der Rohester I ohne Destillation mittels präparativer Mitteldruck-Flüssigkeits-Chromatographie gereinigt wird. Hierbei werden Drucke von 10-20 bar und übliche Adsorbentien, vorzugsweise Kieselgel, eingesetzt.

### Beispiel 1

10 Mol N-(α,α,α-Trifluor-m-tolyl)anthranilsäure(II) werden in 120 Mol 2-(2-Hydroxyethoxy)ethanol(III) suspendiert und unter rühren mit 5 Mol konz. Schwefelsäure versetzt. Der Ansatz wird danach 3 Stunden auf 120 °C erhitzt. Nach dem Abkühlen auf Raumtemperatur wird der Ansatz mit einer Natriumcarbonatlösung (754 g Natriumcarbonat gelöst in 15 Liter Wasser) neutralisiert und 15 Minuten weiter gerührt. Der abgeschiedene Rohester wird mit 1 Liter Chloroform verdünnt, abgetrennt und die wäßrige Lösung noch zweimal mit je 1 Liter Chloroform extrahiert.

Die vereinigten Chloroformphasen werden mit 2 Liter Wasser gewaschen, über Natriumsulfat getrocknet, filtriert und im Vakuum eingedampft. Der erhaltene Rohester wird an einer zweistufigen Kurzwegdestillation destilliert, wobei in der ersten Stufe bei 100 °C und 1,333 Pa (10⁻² Torr) Lösungsmittelreste entfernt werden und in der zweiten Stufe bei 140 °C und 9,33 Pa (7 x 10⁻³ Torr) der reine Ester überdestilliert. Ausbeute 2.563 g (69,4 %).

Der gleiche Ansatz ergab bei 5 Stunden Reaktionszeit und 110 °C Reaktionstemperatur eine Ausbeute von 65,2 %.

### Beispiel 2

1 Mol N-(α,α,α-Trifluor-m-tolyl)anthranilsäure(II) werden zusammen mit 1 Mol p-Toluolsulfonsäure Monohydrat in 10,5 Mol 2-(2-Hydroxyethoxy)ethanol(III) suspendiert und unter Rühren und Stickstoffbegasung 3 Stunden auf 120 °C erhitzt. Nach dem Abkühlen wird der Ansatz unter Rühren durch Zugabe einer gesättigten Natriumcarbonatlösung auf pH 8 gestellt und danach noch 15 Minuten weitergerührt. Der Ansatz wird zweimal mit je 250 ml Chloroform extrahiert und die Chloroformphase mit Wasser gewaschen und über Natriumsulfat getrocknet. Nach dem Eindampfen des Chloroforms wird der erhaltene Rohester einer zweistufigen Kurzwegdestillation unterworfen. Ausbeute 68,6 %.

### Beispiel 3

a) 1 Mol N-(α,α,α-Trifluor-m-tolyl)anthranilsäure(II) werden in 8 Mol 2-(2-Hydroxyethoxy)ethanol(III) suspendiert und nach Zugabe von 0,18 Mol p-Toluolsulfonsäure Monohydrat und 0,23 Mol o-Phosphorsäure (85 %-ig) 6 Stunden auf 120 °C erwärmt.
   Nach dem Abkühlen auf 60 °C wird der Ansatz portionsweise mit 75 g Kaliumcarbonat versetzt und 30 Minuten bei 60 °C weitergerührt. Nach dem Abkühlen auf Raumtemperatur wird der Ansatz zweimal mit je 250 ml Toluol extrahiert. Die mit Wasser gewaschene Toluolphase wird im Vakuum eingedampft und der Rückstand einer zweistufigen Kurzdestillation unterworfen. Ausbeute 40 %.
b) Das Beispiel wurde erneut bei einer Temperatur von 140 °C durchgeführt. Nach einer Reaktionszeit von 6 Stunden wurde eine Ausbeute von 63,2 % erhalten.
c) Beispiel a) wurde mit 0,27 Mol Toluolsulfonsäure und 0,35 Mol Phosphorsäure bei einer Reaktionszeit von 8 Stunden und einer Temperatur von 120 °C wiederholt. Dabei wurde eine Esterausbeute von 56 % erzielt.

### Beispiel 4

32 g Rohester I, hergestellt aus 0,1 Mol II nach dem Verfahren gemäß Beispiel 1 werden in 50 ml einer Mischung aus Toluol und Methanol (95:5) gelöst. Diese Lösung wird auf eine mit Kieselgel 60 (Korngröße 0,04 - 0,063 mm) befüllte Glassäule von 46 cm Länge und einem Durchmessr von 4,9 cm bei einem Druck von 10 - 20 bar mit demselben Lösungsmittel eluiert. Nach dem Abtrennen eines kleinen Vorlaufes, der hauptsächlich Diester enthält, wird das Eluat im Vakuum eingedampft, wobei 25,9 g reiner Ester I erhalten werden, entsprechend einer Gesamtausbeute von 70,2 %.

## Patentansprüche

1. Verfahren zur Herstellung des 2-(2-Hydroxyethoxy)ethanolesters von Flufenaminsäure (N-(α,α,α-Trifluor-mtolyl)anthranilsäure) durch
a) direkte Veresterung von Flufenaminsäure mit einem Überschuß an 2-(2-Hydroxyethoxy)ethanol bei Reaktionstemperaturen von 100 bis 140°C , vorzugsweise 100 bis 120°C in Gegenwart saurer Katalysatoren
b) Entfernung des überschüssigen Diols aus dem Reaktionsansatz und
c) Aufarbeitung des Rohesters
dadurch gekennzeichnet, daß man die Umsetzung mit einem Überschuß an dem Diol von 8-15 Mol je Mol Flufenaminsäure ohne gleichzeitige Entfernung des Reaktionswassers aus dem Reaktionsansatz durchführt und, daß man 0,25 bis 1 Mol Katalysator je Mol Säure einsetzt.

2. Verfahren nach Anspruch 1, dadurch gekennzeichnet, daß man die Umsetzung mit einem Diolüberschuß von 10,5 bis 12 Mol je Mol Flufenaminsäure durchführt.

3. Verfahren nach einem der vorhergehenden Ansprüche dadurch gekennzeichnet, daß man nach Abschluß der Veresterungsreaktion zur Entfernung des überschüssigen Diols dem Reaktionsansatz eine wäßrige Lösung von Alkali- oder Erdalkali-hydroxyd oder -carbonat zusetzt, wobei das rohe Esterprodukt abgetrennt und überschüssiges 2-(2-Hydroxyethoxy)ethanol in die waßrige Phase überführt wird.

4. Verfahren nach Anspruch 1 oder 2, dadurch gekennzeichnet, daß man zur Entfernung des überschüssigen Diols den Reaktionsansatz mit festem Alkali- oder Erdalkali-hydroxyd oder -carbonat bis zur Neutralisation versetzt und anschließend den Rohester mit einem Kohlenwasserstofflösungsmittel, vorzugsweise Toluol, extrahiert.

5. Verfahren nach einem der vorgehenden Ansprüche, dadurch gekennzeichnet, daß man das rohe Esterprodukt durch Mitteldruck-Flüssigkeits-Chromatographie bei Drucken von 10-20 bar unter Einsatz üblicher Adsorbentien, vorzugsweise Kieselgel, reinigt.

6. Verfahren nach einem der vorhergehenden Ansprüche, dadurch gekennzeichnet, daß der Katalysator p-Toluolsulfonsäure-Monohydrat ist.

## Claims

1. A process for the production of the 2-(2-hydroxyethoxy)ethanol ester of flufenamic acid (N-(α,α,α-trifluoro-m-tolyl)anthranilic acid) by
a) direct esterification of flufenamic acid with an excess of 2-(2-hydroxyethoxy)ethanol at reaction temperatures of 100 to 140 °C, preferably 100 to 120 °C, in the presence of acid catalysts,
b) removal of the excess diol from the reaction charge and
c) workup of the crude ester
characterized in that the reaction is carried out with an excess of diol of 8-15 moles per mole flufenamic acid without simultaneous removal of the reaction water from the reaction charge and in that 0.25 to 1 mole catalyst is used per mole acid.

2. A process according to Claim 1, characterized in that the reaction is carried out with an excess of diol of 10.5 to 12 moles per mole flufenamic acid

3. A process according to one of the preceding claims, characterized in that after conclusion of the esterification reaction, for the removal of the excess diol, an aqueous solution of the hydroxide or carbonate of an alkali or alkaline earth metal is added to the reaction charge, after which the crude ester product is separated and excess 2-(2-hydroxyethoxyethanol) is transferred into the aqueous phase.

4. A process according to Claim 1 or 2, characterized in that for the removal of the excess diol the solid hydroxide or carbonate of an alkali or alkaline earth metal is added to the reaction charge as far as neutralization and the crude ester is then extracted with a hydrocarbon solvent, preferably toluene.

5. A process according to one of the preceding claims, characterized in that the crude ester product is purified by medium-pressure liquid chromatography at pressures of 10-20 bar with the use of customary adsorbents, preferably silica gel.

6. A process according to one of the preceding claims, characterized in that the catalyst is p-toluenesulphonic acid monohydrate.

## Revendications

1. Procédé de préparation de l'ester 2-(2-hydroxyéthoxy)éthanolique d'acide flufènamique [acide N-(α,α,α-trifluoro-m-tolyl)anthraniliquel par
a) estérification directe d'acide flufènamique avec un excès de 2-(2-hydroxyéthoxy)éthanol à des températures de réaction de 100 à 140°C, de préférence de 100 à 120°C, en présence de catalyseurs acides,
b) élimination du diol en excès de la charge réactionnelle et
c) traitement ultérieur de l'ester brut,
caractérisé en ce qu'on conduit la réaction avec un excès du diol, de 8 à 15 moles par mole d'acide flufènamique, sans élimination simultanée de l'eau de réaction de la charge réactionnelle, et en ce qu'on utilise 0,25 à 1 mole de catalyseur par mole d'acide.

2. Procédé selon la revendication 1, caractérisé en ce qu'on conduit la réaction avec un excès de diol de 10,5 à 12 moles par mole d'acide flufènamique.

3. Procédé selon la revendication 1 ou 2, caractérisé en ce qu'après l'achèvement de la réaction d'estérification, on ajoute à la charge réactionnelle, pour l'élimination du diol en excès, une solution aqueuse d'hydroxyde ou de carbonate de métal alcalin ou alcalino-terreux, l'ester brut produit étant ainsi séparé et le 2-(2-hydroxyéthoxy)éthanol en excès passant dans la phase aqueuse.

4. Procédé selon la revendication 1 ou 2, caractérisé en ce que, pour l'élimination du diol en excès, on additionne la charge réactionnelle d'hydroxyde ou de carbonate de métal alcalin ou alcalino-terreux jusqu'à la neutralisation, puis on extrait l'ester brut avec un solvant hydrocarboné, de préférence le toluène.

5. Procédé selon l'une quelconque des revendications 1 à 4, caractérisé en ce qu'on purifie l'ester brut produit par chromatographie liquide sous pression moyenne, sous des pressions de 10-20 bar, en utilisant des substances adsorbantes usuelles, de préférence du gel de silice.

6. Procédé selon l'une quelconque des revendications 1 à 5, caractérisé en ce que le catalyseur est l'acide p-toluènesulfonique monohydraté.
